# EUROPEAN PATENT APPLICATION

(11) **EP 4 252 739 A1**
(43) Date of publication of application: **04.10.2023**
(21) Application number: 21898317.9
(22) Date of filing: 28.09.2021
(51) Int. Cl.: A61K 8/895, A61K 8/31, A61Q 19/00

(54) **COSMETIC COMPOSITION AND PREPARATION METHOD THEREFOR**

(30) Priority: 26.11.2020 KR 20200161404
(71) Applicant: KCC Silicone Corporation, Seoul 06608 (KR)
(72) Inventor: KO, Tae Ho, Yongin-si, Gyeonggi-do 17003 (KR); LEE, Kwang Hun, Suwon-si, Gyeonggi-do 16563 (KR); JUNG, Yu Suk, Seoul 06276 (KR)
(74) Representative: Cabinet Laurent & Charras
(86) International application number: PCT/KR2021/013252
(87) International publication number: WO 2022/114490

(57) **Abstract**

The present invention provides a cosmetic composition and a preparation method therefor, the composition comprising an acryl-silicone copolymer and a diluent, wherein the diluent is a naturally occurring hydrocarbon, and the composition is prepared by removing an organic solvent through vacuum distillation carried out at a pressure of 1 torr to 500 torr and a temperature of 30°C to 200°C under the presence of the naturally occurring hydrocarbon.

## Description

### [Technical Field]

The present invention relates to a cosmetic composition that is less harmful to the human body by using a naturally occurring diluent and allows stable film formation, and a preparation method therefor.

### [Background Art]

Silicone has excellent stability, feel of use and the like, and is used as a raw material in various fields. Particularly, silicone used in a cosmetic composition is a resin that is soft and has excellent lubricity like a dimethylpolysiloxane polymer. In addition, a cosmetic composition for hair commonly includes silicone as described above and silicone oil as a diluent. Examples of the silicone oil may include low molecular siloxane-based compounds such as cyclopentasiloxane, isododecane, dimethicone and the like.

Specifically, Korean Patent Application Laid-Open Publication No. 2016-0129594 (Patent Document 1) discloses a composition for hair drying, the composition including volatile silicone oil, ethanol and a silicone resin. However, there are increasing demands for silicone having more enhanced feel of use and less harmful to the human body by using a naturally occurring raw material in the cosmetic industry recently. Accordingly, chemical materials such as volatile silicone oil of Patent Document 1 are excluded from raw materials of a cosmetic composition due to their harmfulness to the human body.

As an alternative thereto, Korean Patent Application Laid-Open Publication No. 2019-0121408 (Patent Document 2) discloses a transfer-resistant cosmetic composition prepared using a method including a) forming a premix by dissolving a silicone acrylate polymer powder in a volatile solvent; and b) mixing the premix with a carrier. However, existing acrylate polymers such as Patent Document 2 are generally synthesized in a solvent, and have a problem of needing to go through a powdering process.

Accordingly, there are needs for research and development on a method for preparing a cosmetic composition having an excellent film forming property and hardly harmful to the human body by using a naturally occurring material as a diluent, and a cosmetic composition prepared using the same.

### [Disclosure of the Invention]

### [Technical Problem]

In view of the above, the present invention is directed to providing a method for preparing a cosmetic composition that has an excellent film forming property and is hardly harmful to the human body by using a naturally occurring material as a diluent, and a cosmetic composition prepared using the same.

### [Technical Solution]

The present invention provides a cosmetic composition including an acryl-silicone copolymer and a diluent, wherein the diluent is a naturally occurring hydrocarbon, and the composition is prepared by removing an organic solvent through vacuum distillation carried out at a pressure of 1 torr to 500 torr and a temperature of 30°C to 200°C under the presence of the naturally occurring hydrocarbon.

In addition, the present invention provides a method for preparing a cosmetic composition, the method including preparing an acryl-silicone copolymer by reacting polysiloxane and an acrylate-based compound in an organic solvent; and
mixing the acryl-silicone copolymer and a diluent, and removing the organic solvent through vacuum distillation,
wherein the vacuum distillation is carried out at a pressure of 1 torr to 500 torr and a temperature of 30°C to 200°C under the presence of the diluent, and
the diluent is a naturally occurring hydrocarbon.

### [Advantageous Effects]

A cosmetic composition according to the present invention uses a naturally occurring hydrocarbon as a diluent, thereby less harmful to the human body. In addition, a method for preparing a cosmetic composition according to the present invention is capable of preparing a cosmetic composition that is less harmful to the human body by using a naturally occurring hydrocarbon as a diluent while effectively removing an organic solvent in the composition. In addition, the preparation method is excellent in process costs and thereby has high economic feasibility, and is effective in reducing a loss of the naturally occurring hydrocarbon, that is, a natural oil, by vacuum distillation.

In addition, a cosmetic composition prepared using the preparation method includes a naturally occurring hydrocarbon as a diluent, and therefore, is less harmful to the human body and has an excellent a film forming property, and therefore, is very suitable as a carrier.

### [Best Mode]

Hereinafter, the present invention will be described in detail.

A "weight average molecular weight" used in the present specification is measured using a common method known in the art, and for example, may be measured using a GPC (gel permeation chromatography) method.

In addition, in the present specification, "(meth)acryl" means "acryl" and/or "methacryl", and "(meth)acrylate" means "acrylate" and/or "methacrylate".

### Method for Preparing Cosmetic Composition

A method for preparing a cosmetic composition according to the present invention includes preparing an acryl-silicone copolymer; and mixing the acryl-silicone copolymer and a diluent, and removing an organic solvent through vacuum distillation.

### Preparation of Acryl-Silicone Copolymer

In this step, an acryl-silicone copolymer is prepared by reacting polysiloxane and an acrylate-based compound in an organic solvent.

The polysiloxane may be (meth)acryloxyalkyl polydimethylsiloxane, and for example, may be represented by the following Chemical Formula 1.

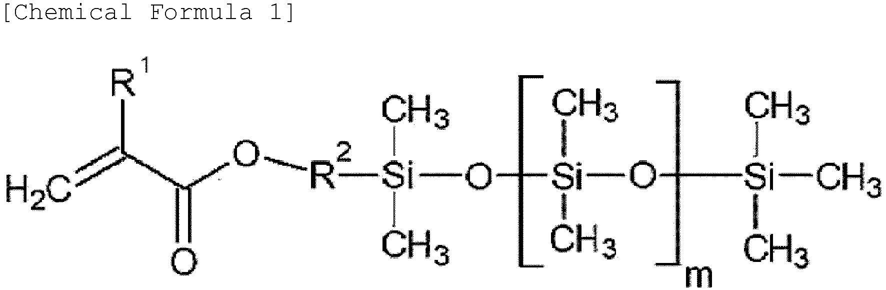

In Chemical Formula 1, R¹ is a Ci-io alkyl group or a hydrogen group,
R² is a C₁₋₁₀ alkylene group, and
m is an integer of 10 to 30.

For example, R¹ may be a methyl group or a hydrogen group, R² may be a C₁₋₅ alkylene group, and m may be an integer of 15 to 30.

Herein, the alkyl group and the alkylene group may be linear or branched. In addition, the alkylene group refers to a branched, linear or cyclic divalent radical derived by removing one hydrogen atom from a carbon atom of an alkyl group. For example, the alkylene group may be methylene (-CH₂-), 1,1-ethylene (-CH(CH₃)-), 1,2-ethylene (-CH₂CH₂-), 1,1-propylene (-CH(CH₂CH₃)-) , 1,2-propylene (-CH₂CH(CH₃)-), 1,3-propylene (-CH₂CH₂CH₂-), 1,4-butylene (-CH₂CH₂CH₂CH₂-), 2,4-butylene (-CH₂(CH₃)CH₂CH₂-), 1,6-hexylene (-CH₂CH₂CH₂CH₂CH₂CH₂-) or the like, but is not limited thereto.

In addition, having m in the above-mentioned range is effective in enhancing softness and feel of use of the composition.

The acrylate-based compound may be a compound including one or more types selected from the group consisting of acrylates and methacrylates, that is, an acrylate group and/or a methacrylate group. Specifically, the acrylate-based compound may be one or more types selected from the group consisting of C_{1~10} alkyl (meth) acrylates and hydroxy C_{1~10} alkyl (meth) acrylates. For example, the acrylate-based compound may be a C_{1~10} alkyl methacrylate such as methyl methacrylate, ethyl methacrylate, butyl methacrylate or 2-ethylhexyl methacrylate; a hydroxy C_{1~10} alkyl methacrylate such as 2-hydroxyethyl methacrylate or 2-hydroxypropyl methacrylate; a C_{1~10} alkyl acrylate such as methyl acrylate, ethyl acrylate, butyl acrylate or 2-ethylhexyl acrylate; or a hydroxy C_{1~10} alkyl acrylate such as 2-hydroxyethyl acrylate or 2-hydroxypropyl acrylate.

In addition, the organic solvent is not particularly limited as long as it is commonly usable in preparing a silicone copolymer, and for example, may include one or more types selected from the group consisting of aromatic compounds such as xylene and toluene; aliphatic hydrocarbon compounds such as isododecane, undecane and tridecane; and polar solvents such as butyl acetate. In addition, commercially available products of the organic solvent may include, for example, Isopar C, E, G, H and the like.

An initiator may be added when reacting the polysiloxane and the acrylate-based compound. Herein, a common radical polymerization reaction initiator may be used as the initiator, and examples thereof may include tert-butyl peroxy-2-ethylhexanoate and the like.

The polysiloxane and the acrylate-based compound may be reacted in a weight ratio of 1: 0.2 to 5 or a weight ratio of 1: 0.5 to 2.5. When the weight ratio is less than the above-mentioned range in the reaction between the polysiloxane the acrylate-based compound, that is, when a small amount of the acrylate-based compound is used, a film-type dry film is not formed, resulting in a problem of not functioning as a film former, and when the weight ratio is greater than the above-mentioned range, that is, when the acrylate-based compound is used in excess, a problem of the cured material becoming a hard material like plastic and not being dissolved in a solvent may occur.

In addition, the polysiloxane and the acrylate-based compound may be reacted until the content of the unreacted acrylate group becomes 0.1% by weight to 5% by weight or 0.5% by weight to 2% by weight at a reaction temperature of 50°C to 180°C or 70°C to 150°C.

The acryl-silicone copolymer prepared by the reaction as described above may have a structure in which a siloxane pendant is added to the acryl main chain, which has advantages of having water repellency and oil repellency, a property of siloxane, and forming a film with excellent gloss, a property of an acryl group. Specifically, the acryl-silicone copolymer may be represented by the following Chemical Formula 2.

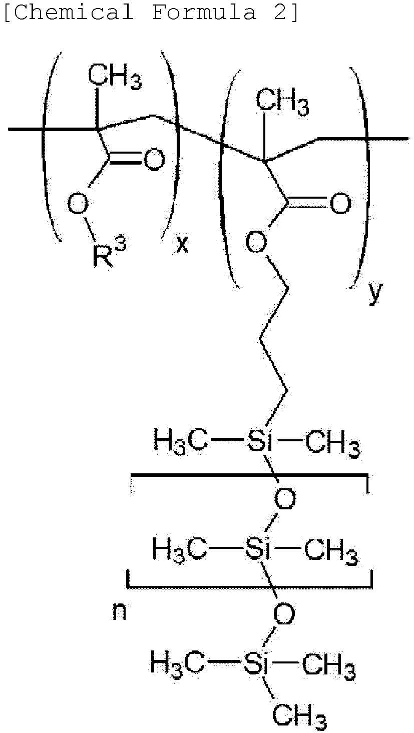

In Chemical Formula 2, R³ is a C_{1~10} hydrocarbon group, x is an integer of 3 to 20, y is an integer of 3 to 25, and n is an integer of 10 to 30.

Specifically, R³ is a C_{1~10} alkyl group, x is an integer of 5 to 20, y is an integer of 5 to 20, n is an integer of 15 to 30, and the alkyl group may be substituted with a hydroxyl group or a halogen atom. Herein, the alkyl group may be linear or branched.

Having x, y and n in the above-mentioned ranges is effective in providing film feel to the composition and enhancing durability and feel of use.

In addition, the acryl-silicone copolymer has a glass transition temperature (Tg) of 35°C to 60°C, 37°C to 55°C or 38°C to 50°C, and may have a silicone content of 65% by weight to 90% by weight, 70% by weight to 88% by weight or 75% by weight to 85% by weight based on the total weight of the acryl-silicone copolymer. The acryl-silicone copolymer having a glass transition temperature in the above-mentioned range is effective in enhancing a film forming property and film durability, and having the silicone content in the above-mentioned range is effective in increasing feel of use.

The acryl-silicone copolymer has a weight average molecular weight (Mw) of 7,000 g/mol to 48,000 g/mol, 8,000 g/mol to 45,000 g/mol or 10,000 g/mol to 45,000 g/mol, and may have viscosity of 100 cP to 700 cP, 150 cP to 650 cP or 200 cP to 600 cP at 25°C. When the acryl-silicone copolymer has a weight average molecular weight (Mw) in the above-mentioned range, the film has excellent durability and glossiness after drying, and having viscosity in the above-mentioned range at 25°C is effective in obtaining excellent film feel of the composition.

### Removing Organic Solvent

In this step, the acryl-silicone copolymer and a diluent are mixed, and vacuum distilled to remove the organic solvent. Through this, a cosmetic composition less harmful to the human body may be prepared by removing the organic solvent in the acryl-silicone copolymer. Herein, the organic solvent is a solvent used when preparing the acryl-silicone copolymer, and has an odor or a bad smell.

The diluent is a naturally occurring hydrocarbon, and for example, may be a naturally occurring hydrocarbon compound having 9 to 15 carbon atoms or 12 to 15 carbon atoms. Specifically, the diluent may be an alkane having 10 to 14 carbon atoms, and more specifically, a mixture of undecane and tridecane. Specifically, the diluent may include undecane and tridecane in a weight ratio of 1:0.1 to 1:3 or a weight ratio of 1:1 to 1:2.5.

When the mixed weight ratio of undecane and tridecane is less than the above-mentioned range, that is, when undecane having a low carbon number is mixed in excess, the undecane vaporizes during vacuum distillation causing a problem of increasing the amount of waste, and when the mixed weight ratio is greater than the above-mentioned range, that is, when tridecane having a high carbon number is mixed in excess, the amount of waste is reduced, however, volatility is poor when applied to cosmetics causing a problem of insufficient film feel.

The acryl-silicone copolymer and the diluent may be mixed in a weight ratio of 1: 0.1 to 10, a weight ratio of 1:0.3 to 5 or a weight ratio of 1: 0.5 to 2. When the mixed weight ratio of the acryl-silicone copolymer and the diluent is less than the above-mentioned range, that is, when a small amount of the diluent is mixed, a problem such that local reactions occur during the reaction or it is difficult to maintain the reactor temperature may occur, and when the mixed weight ratio is greater than the above-mentioned range, that is, when the diluent is mixed in excess, the excess diluent may cause problems of increasing raw material costs and lowering productivity.

The vacuum distillation may be carried out at a pressure of 1 torr to 500 torr and a temperature of 30°C to 200°C. For example, the vacuum distillation may be carried out at a pressure of 1 torr to 200 torr, 4 torr to 50 torr or 5 torr to 30 torr and a temperature of 40°C to 150°C or 60°C to 120°C. When the pressure during vacuum distillation is less than the above-mentioned range, removing the organic solvent is insufficient, and when the pressure is greater than the above-mentioned range, a disposed amount of the diluent increases causing a problem of lowering economic feasibility. In addition, when the temperature during vacuum distillation is less than the above-mentioned range, a problem of insufficient removal of the organic solvent, a distillate, may occur, and when the temperature is greater than the above-mentioned range, much energy needs to be consumed due to too high temperature in the distillation, and the acryl-silicone copolymer may be decomposed due to high temperature treatment.

In addition, in order to minimize a disposed amount of the diluent, a process of nitrogen bubbling may be further included during the vacuum distillation. In other words, in this step, the acryl-silicone copolymer and the diluent are mixed, and the organic solvent may be removed by nitrogen bubbling during vacuum distillation.

The cosmetic composition from which the organic solvent is removed through vacuum distillation as described above may include the organic solvent in a content of less than 0.2% by weight, 0.0001% by weight to 0.2% by weight or 0.0001% by weight to 0.1% by weight with respect to the total composition weight. When the content of the organic solvent is greater than the above-mentioned range, a smell caused by the organic solvent, that is, a bad smell or an odor, may occur in the product and skin irritation may be caused. Herein, the content of the organic solvent is a content of the solvent excluding the naturally occurring hydrocarbon that is the diluent.

The method for preparing a cosmetic composition according to the present invention as described above is capable of preparing a cosmetic composition that is less harmful to the human body by using a naturally occurring hydrocarbon as a diluent while effectively removing an organic solvent in the composition. In addition, the preparation method is excellent in process costs and thereby has high economic feasibility, and is effective in reducing a loss of the naturally occurring hydrocarbon, that is, a natural oil, by vacuum distillation.

### Cosmetic Composition

The cosmetic composition according to the present invention includes an acryl-silicone copolymer and a diluent, the diluent is a naturally occurring hydrocarbon, and the composition is prepared by removing an organic solvent through vacuum distillation carried out at a pressure of 1 torr to 500 torr and a temperature of 30°C to 200°C under the presence of the naturally occurring hydrocarbon.

In addition, the cosmetic composition may include the organic solvent in a content of less than 0.2% by weight, 0.0001% by weight to 0.2% by weight or 0.0001% by weight to 0.1% by weight with respect to the total composition weight. When the content of the organic solvent is greater than the above-mentioned range, a bad smell or an odor, a smell of the organic solvent, may occur in the product and skin irritation may be caused. Herein, the content of the organic solvent is a content of the solvent excluding the naturally occurring hydrocarbon that is the diluent.

The diluent is as defined in the preparation method. For example, the cosmetic composition may include undecane and tridecane in a weight ratio 1:0.1 to 1:3 or a weight ratio 1:1 to 1:2.5.

In addition, in the cosmetic composition, the acryl-silicone copolymer and the diluent may be mixed in a weight ratio of 1: 0.1 to 10, a weight ratio of 1: 0.3 to 5 or a weight ratio of 1: 0.5 to 2.

In addition, the cosmetic composition may have a solid content of 20% by weight to 50% by weight or 30% by weight to 45% by weight with respect to the total composition weight, and may have viscosity of 100 cP to 6,000 cP, 400 cP to 4,000 cP or cP 500 to 3,000 cP at 25°C.

By including a naturally occurring hydrocarbon as the diluent, the cosmetic composition as described above is less harmful to the human body, and has an excellent film forming property, and therefore, is very suitable as a carrier.

Hereinafter, the present invention will be described more specifically with respect to the following examples. However, descriptions by the following examples are only intended to specifically describe specific embodiments of the present invention, and are not intended to limit or limitedly interpret the scope of right of the present invention to the descriptions provided in these examples.

### Example 1. Preparation of Cosmetic Composition

In a reaction vessel, 50 parts by weight of polysiloxane, 15 parts by weight of butyl methacrylate, 15 parts by weight of 2-ethylhexyl acrylate, 20 parts by weight of methyl methacrylate, and 100 parts by weight of Isopar E (product of ExxonMobil, boiling point: 114°C to 136°C) as an organic solvent were introduced, and the mixture was reacted at 100°C until viscosity thereof becomes 500 cP to prepare an acryl-silicone copolymer. Herein, methacryloxypropyl polydimethylsiloxane was used as the polysiloxane, and the prepared acryl-silicone copolymer has a glass transition temperature 50°C, a weight average molecular weight of 30,000 g/mol, and viscosity of 500 cP at 25°C.

After that, 100 parts by weight of the acryl-silicone copolymer and 150 parts by weight of a mixture of undecane and tridecane (including undecane and tridecane in a weight ratio of 1:2.3) as a diluent were mixed, and the result was vacuum distilled for 3 hours at a pressure of 10 torr and a temperature of 95°C to remove the Isopar E that is the organic solvent, and then distilled for 5 hours at a pressure of 10 torr and a temperature of 95°C under nitrogen gas bubbling to prepare a cosmetic composition.

The prepared cosmetic composition had an undecane and tridecane mixture content of 60% by weight with respect to the total composition weight, had viscosity of 715 cP at 25°C, and had a solid content of 40% by weight with respect to the total composition weight.

### Examples 2 to 10 and Comparative Examples 1 to 5.

Cosmetic compositions were prepared in the same manner as in Example 1 except that vacuum distillation or distillation was carried out under the same conditions as described in Table 1, and the type of the diluent was changed.

Herein, vacuum distillation was carried out without nitrogen gas bubbling in Example 8.

**[Table 1]**

| Category | Distillation Condition | | | Diluent |
|---|---|---|---|---|
| | Pressure (torr) | Temperature (°C) | Time (Hour) | |
| | | | | |
| Example 1 | 10 | 95 | 8 | Include undecane and tridecane in weight ratio of 1: 2.3 |
| Example 2 | 50 | 95 | 8 | Include undecane and tridecane in weight ratio of 1: 2.8 |
| Example 3 | 4 | 95 | 8 | Include undecane and tridecane in weight ratio of 1: 1.3 |
| Example 4 | 10 | 95 | 8 | Include undecane and tridecane in weight ratio of 1: 0.5 |
| Example 5 | 10 | 130 | 8 | Include undecane and tridecane in weight ratio of 1: 2.3 |
| Example 6 | 10 | 35 | 8 | Include undecane and tridecane in weight ratio of 1: 2.3 |
| Example 7 | 10 | 95 | 8 | Include undecane and tridecane in weight ratio of 1: 3.2 |
| Example 8 | 10 | 95 | 8 | Include undecane and tridecane in weight ratio of 1: 2.3 |
| Example 9 | 10 | 95 | 8 | Include undecane and tridecane in weight ratio of 1: 0.05 |
| Example 10 | 10 | 95 | 5 | Include pentadecane and tridecane in weight ratio of 1: 2.3 |
| Comparative Example 1 | 760 | 95 | 2 | Include undecane and tridecane in weight ratio of 1: 2.3 |
| Comparative Example 2 | 10 | 95 | 2 | Decamethylcyclopentasiloxane |
| Comparative Example 3 | 0.5 | 95 | 5 | Include undecane and tridecane in weight ratio of 1: 2.3 |
| Comparative Example 4 | 10 | 160 | 5 | Include undecane and tridecane in weight ratio of 1: 2.3 |
| Comparative Example 5 | 520 | 95 | 5 | Include undecane and tridecane in weight ratio of 1: 2.3 |

### Test Example. Evaluation on Properties

Properties of the cosmetic compositions of the examples and the comparative examples were measured in the following manner, and the results are shown in Table 2.

### (1) Viscosity

Viscosity at 25°C was measured using a viscometer (HBDV Brookfield viscometer, spindle #93, 2.5 rpm).

### (2) Appearance

Transparency was observed by visually observing the appearance at 25°C after removing bubbles in the cosmetic composition. As for the evaluation result, the transparency was relatively evaluated in 4 stages of transparent, slightly transparent, slightly opaque and opaque, and the color was visually identified.

### (3) Content of Organic Solvent and Diluent

The content of Isopar E that is the organic solvent and the content of the diluent in the cosmetic composition were measured by gas chromatography.

### (4) Film Feel

After placing 0.20 g of the cosmetic composition on the back of a hand and spreading it thinly as if drawing a circle, feel of use was evaluated through feel during the application and skin sensation. Specifically, whether a film was formed and stickiness is less was compared, and as for the evaluation result herein, the film feel was relatively evaluated in 5 stages of very good, good, fair, insufficient and very insufficient.

### (5) Disposed Amount of Diluent

The difference in the diluent weight after vacuum distillation based on the diluent weight before vacuum distillation was calculated as a disposed amount of the diluent.

**[Table 2]**

| | Viscosity at 25°C (cP) | Appearance | Content of Organic Solvent | Film Feel | Disposed Amount of Diluent (Natural Solvent) (% by weight) |
|---|---|---|---|---|---|
| Example 1 | 715 | Transparent, Colorless | <5 ppm | Very Good | 2 |
| Example 2 | 210 | Transparent, Colorless | 20 ppm | Good | 3 |
| Example 3 | 2,525 | Transparent, Colorless | <5 ppm | Very Good | 22 |
| Example 4 | 2, 987 | Transparent, Colorless | <5 ppm | Good | 37 |
| Example 5 | 5,187 | Transparent, Light Yellow | <5 ppm | Good | 48 |
| Example 6 | 180 | Transparent, Light Yellow | 1,200 ppm | Good | 5 |
| Example 7 | 2,201 | Transparent, Colorless | 108 ppm | Good | 45 |
| Example 8 | 628 | Transparent, Colorless | 258 ppm | Good | 11 |
| Example 9 | 25,101 | Transparent, Colorless | <5 ppm | Fair | 61 |
| Example 10 | 157 | Transparent, Colorless | <5 ppm | Fair | 3 |
| Comparative Example 1 | 57 | Transparent, Colorless | 3,000 ppm | Very Insuffi cient | 1 |
| Comparative Example 2 | 578 | Transparent, Colorless | 628 ppm | Insuffi cient | 21 |
| Comparative Example 3 | 7,802 | Transparent, Colorless | <5 ppm | Insuffi cient | 30 |
| Comparative Example 4 | 82,130 | Transparent, Colorless | <5 ppm | Insuffi cient | 60 |
| Comparative Example 5 | 320 | Transparent, Colorless | 1,200 ppm | Insuffi cient | 2 |

As shown in Table 2, the compositions of the examples had proper viscosity, were colorless and transparent in the appearances, were less harmful to the human body due to the low content of the organic solvent having a smell (bad smell or odor), and had excellent film feel.

On the other hand, Comparative Example 1 subjected to simple distillation instead of vacuum distillation had a high content of the organic solvent, had low viscosity at 25°C, and had insufficient film feel.

In addition, Comparative Example 2 including decamethylcyclopentasiloxane (D5) instead of a naturally occurring hydrocarbon as the diluent had a smell (bad smell and/or odor) caused by D5, and had insufficient film feel.

Furthermore, Comparative Example 3 having an excessively low pressure during distillation and Comparative Example 5 having an excessively high pressure during distillation had insufficient film feel.

In addition, Comparative Example 4 having a high temperature during distillation had high viscosity at 25°C and had insufficient film feel.

## Claims

1. A cosmetic composition, comprising:
an acryl-silicone copolymer; and
a diluent;
wherein the diluent is a naturally occurring hydrocarbon, and
the composition is prepared by removing an organic solvent through vacuum distillation carried out at a pressure of 1 torr to 500 torr and a temperature of 30°C to 200°C under the presence of the naturally occurring hydrocarbon.

2. The cosmetic composition of claim 1, wherein the diluent comprises undecane and tridecane in a weight ratio of 1: 0.1 to 1: 3.

3. The cosmetic composition of claim 1, which comprises the acryl-silicone copolymer and the diluent in a weight ratio of 1: 0.1 to 10.

4. A method for preparing a cosmetic composition, the method comprising:
preparing an acryl-silicone copolymer by reacting polysiloxane and an acrylate-based compound in an organic solvent; and
mixing the acryl-silicone copolymer and a diluent, and removing the organic solvent through vacuum distillation,
wherein the vacuum distillation is carried out at a pressure of 1 torr to 500 torr and a temperature of 30°C to 200°C under the presence of the diluent; and
the diluent is a naturally occurring hydrocarbon.

5. The method for preparing a cosmetic composition of claim 4, wherein the vacuum distillation is carried out at a pressure of 1 torr to 200 torr and a temperature of 40°C to 150°C.

6. The method for preparing a cosmetic composition of claim 4, wherein the diluent is a naturally occurring hydrocarbon compound having 9 to 15 carbon atoms.

7. The method for preparing a cosmetic composition of claim 4, wherein the diluent includes undecane and tridecane in a weight ratio of 1: 0.1 to 1: 3.
